# EUROPEAN PATENT SPECIFICATION

(11) **EP 0 465 009 B1**
(45) Date of publication and mention of the grant of the patent: **09.08.2000**
(21) Application number: 91305064.7
(22) Date of filing: 05.06.1991
(51) Int. Cl.: C12N 15/29, C12N 15/82, A01N 65/00, C12N 5/10, C12N 1/21, A01H 5/00

(54) **Antimicrobial peptides and plant disease resistance based thereon**
Antimikrobielle Peptide und darauf basierte Pflanzenkrankheitsresistenz
Peptides antimicrobiens et résistance aux maladies de plantes basé sur ceux-ci

(30) Priority: 05.06.1990 US 536127
(43) Date of publication of application: 08.01.1992
(73) Proprietor: PIONEER HI-BRED INTERNATIONAL, INC., Des Moines, Iowa 50309 (US)
(72) Inventor: Duvick, Jonathan P., Des Moines,Polk County 50319 (US); Rood, Tracy A., Polk County, Iowa (US); Rao, Aragula G., Polk County, Iowa 50322 (US)
(74) Representative: Goldin, Douglas Michael

(56) References cited:
- WO-A-89/02744
- WO-A-89/04371
- WO-A-90/03725
- THE FASEB JOURNAL, vol. 4, no. 7, 26th April 1990, page A2165 abstract no. 2730; J. DUVICK et al.: "Purification and characterization of an anti-microbial peptide from maize kernels"
- PHYTOPATHOLOGY, vol. 80, no. 10, 4th-8th August 1990, page 1053, abstract no. A771; J. DUVICK et al.: "Purification and characterization of peptides from maize seed which show antifungal activity"
- THE EMBO JOURNAL, vol. 7, no. 6, 1988, pages 1559-1565, IRL Press Ltd, Oxford, GB; H. BOHLMANN et al.: "Leaf-specific thionins of barley - a novel class of cell wall proteins toxic to plant-pathogenic fungi and possibly involved in the defense mechanism of plants"

## Description

### Technical Field

This invention relates to materials and methods for killing fungi and other microorganisms which are harmful to plants, and materials and methods for imparting disease resistance to plants.

### Background of the Invention

Numerous fungi and bacteria are serious pests of common agricultural crops. One method of controlling diseases has been to apply antimicrobial organic or semiorganic chemicals to crops. This method has numerous, art-recognized problems. A more recent method of control of microorganism pests has been the use of biological control organisms which are typically natural competitors or inhibitors of the troublesome microorganisms. However, it is difficult to apply biological control organisms to large areas, and even more difficult to cause those living organisms to remain in the treated area for an extended period. Still more recently, techniques in recombinant DNA have provided the opportunity to insert into plant cells cloned genes which express antimicrobial compounds. This technology has given rise to additional concerns about eventual microbial resistance to well-known, naturally occurring antimicrobials, particularly in the face of heavy selection pressure, which may occur in some areas. Thus, a continuing need exists to identify naturally occurring antimicrobial compounds which can be formed by plant cells directly by translation of a single structural gene.

European Patent Application 204,590, based upon U.S. Patent application Serial No. 725,368, describes a method of genetically modifying a plant cell to control expression of heterologous foreign structure genes. In the method, the plant cell is transformed to contain a pRi T-DNA promoter and a heterologous foreign structural gene, the promoter and the structural gene being in such position and orientation with respect to one another that the structural gene is expressible in a plant cell under control of the promoter.

Likewise, European Patent Application 186,425, based upon U.S. patent application Serial No. 685,824, describes a recombinant DNA expression vector which comprises (a) a transcription unit, flanked by T-DNA border sequences, which comprises a promoter and associated amino terminal region encoding sequences and a terminator signal sequence in which the sequences are derived from one or more genes which are naturally expressed in a plant cell, and (b) an antibiotic resistance gene-encoding sequence located between the promoter and associated amino-terminal region-encoding sequence and the terminator sequence and (c) a DNA fragment containing a replicon that is functional in Agrobacterium.

PCT application 8807087, based upon U.S. patent application 168,109, discloses a recombinant virus expression system comprising a Heliothis polyhedrin promoter and a nucleotide sequence encoding a heterologous peptide or protein, which may have antimicrobial activity.

Duvick et al., Abstract No. 2730, The FASEB Journal 4 (7) (1990) discloses isolation of a small, basic maize seed peptide, CMIII, and that CMIII has potent inhibitory activity against several plant pathogenic fungi, including seed pathogens of maize. CMIII is particularly active against the plant pathogenic fungus Sclerotinia, and also against the maize seed pathogen, Fusarium graminearum.

### Brief Description of the Drawings

Figure 1 is a graph showing the results of cellulose chromatography of crude plant extract as described in the examples 1 & 2.
Figure 2 is a graph illustrating the results of FPLC purification of crude plant extract as described in Examples 1 & 2.
Figure 3 is a graph illustrating the data from a single microbial inhibitation experiment. Inhibition was scored relative to control wells containing no peptide, as described in Example 5.
Figures 4, 5 & 6 illustrate the CD, absorption and flourescence spectra, respectively, of CM-III.

A DNA sequence which codes for CMIII has now been isolated, cloned and inserted into an appropriate expression cassette for introduction into cells of a plant. Thus in one aspect, the present invention is a method for killing or inhibiting a plant pathogenic microorganism which is susceptible to the antimicrobial polypeptide CMIII having the amino acid sequence Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly, comprising providing plants having inserted into their genome a DNA sequence coding for CMIII, in proper reading frame relative to transcription initiator and promoter sequences active in the plant, so as to cause expression of an antimicrobial amount of CMIII in tissues of the plant which are normally infected by said pathogen.

Plants for the purpose of the present invention are preferably plants susceptible to infection and damage by one or more of Fusarium graminearum, Fusarium moniliforme, Aspergillus flavus, Alternaria longipes, Sclerotinia sclerotiorum, and Sclerotinia trifoliorum. These include corn (Zea mays) and sorghum (Sorghum bicolor). However, this is not to be construed as limiting, inasmuch as these species are among the most difficult commercial crops to reliably transform and regenerate, and these microorganisms also infect certain other crops. Thus the methods of this invention are readily applicable via conventional techniques to numerous plant species, if they are found to be susceptible to the plant pests listed hereinabove, including, without limitation, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersionn, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Triticum, and Datura.

Preferred plants that are to be transformed according to the methods of this invention are cereal crops, including maize, rye, barley, wheat, sorghum, oats, millet, rice, triticale, sunflower, alfalfa, rape seed and soybean.

In general, since the object of the invention is to confer resistance to a microorganism to which the plant is susceptible, in some cases CMIII will not be native to the plant, i.e., the CMIII gene will come from a species other than the plant being transformed. However, in species which produce CMIII but in lower than antimicrobial amounts, it may be preferable to insert a gene for CMIII under strong constitutive promoter control to cause overproduction of CMIII, thus achieving antimicrobial levels and conferring effective disease resistance. Alternatively, where a plant produces CMIII but the CMIII is not produced in or not distributed to tissues which are normally affected by the disease, a tissue specific promoter can be used to provide localized expression or overproduction of CMIII. A tissue specific promoter can be used in any instance where it may be desirable to localize production of CMIII to an infected tissue or to a tissue which is efficient in production of CMIII.

A DNA sequence which codes for CMIII can be obtained, for example, by screening a maize genomic or cDNA library with oligonucleotide probes as described in Example 3 and introduced into a selected plant expression cassette. Alternatively, on the basis of the amino acid sequence for CMIII provided herein, a synthetic DNA sequence coding for CMIII can be prepared using known methods and inserted into an appropriate plant expression cassette.

Numerous plant expression cassettes and vectors are well known in the art. By the term "expression cassette" is meant a complete set of control sequences including initiation, promoter and termination sequences which function in a plant cell when they flank a structural gene in the proper reading frame. Expression cassettes frequently and preferably contain an assortment of restriction sites suitable for cleavage and insertion of any desired structural gene. It is important that the cloned gene have a start codon in the correct reading frame for the structural sequence. In addition, the plant expression cassette preferably includes a strong constitutive promoter sequence at one end to cause the gene to be transcribed at a high frequency, and a poly-A recognition sequence at the other end for proper processing and transport of the messenger RNA. An example of such a preferred (empty) expression cassette into which the cDNA of the present invention can be inserted is the pPHI414 plasmid developed by Beach et al. of Pioneer Hi-Bred International, Inc., Johnston, IA. Highly preferred plant expression cassettes will be designed to include one or more selectable marker genes, such as kanamycin resistance or herbicide tolerance genes.

By the term "vector" herein is meant a DNA sequence which is able to replicate and express a foreign gene in a host cell. Typically, the vector has one or more endonuclease recognition sites which may be cut in a predictable fashion by use of the appropriate enzyme. Such vectors are preferably constructed to include additional structural gene sequences imparting antibiotic or herbicide resistance, which then serve as markers to identify and separate transformed cells. Preferred markers/selection agents include kanamycin, chlorosulfuron, phosphonothricin, hygromycin and methotrexate. A cell in which the foreign genetic material in a vector is functionally expressed has been "transformed" by the vector and is referred to as a "transformant."

A particularly preferred vector is a plasmid, by which is meant a circular double-stranded DNA molecule which is not a part of the chromosomes of the cell.

As mentioned above, both genomic and cDNA encoding CMIII may be used in this invention. The vector of interest may also be constructed partially from a cDNA clone and partially from a genomic clone. When the gene of interest has been isolated, genetic constructs are made which contain the necessary regulatory sequences to provide for efficient expression of the gene in the host cell. According to this invention, the genetic construct will contain (a) a first genetic sequence coding for CMIII and (b) one or more regulatory sequences operably linked on either side of the structural gene of interest. Typically, the regulatory sequences will be selected from the group comprising of promoters and terminators. The regulatory sequences may be from autologous or heterologous sources.

Promoters that may be used in the genetic sequence include nos, ocs and CaMV promoters.

An efficient plant promoter that may be used is an overproducing plant promoter. Overproducing plant promoters that may be used in this invention include the promoter of the small sub-unit (ss) of the ribulose-1,5-biphosphate carboxylase from soybean (Berry-Lowe et al, J. Molecular and App. Gen., 1:483-498 (1982)), and the promoter of the cholorophyll a-b binding protein. These two promoters are known to be light-induced, in eukaryotic plant cells (see, for example, Genetic Engineering of Plants, An Agricultural Perspective, A. Cashmore, Pelham, New York, 1983, pp. 29-38, G. Coruzzi et al., J. Biol. Chem., 258:1399 (1983), and P. Dunsmuir, et al., J. Molecular and App. Gen., 2:285 (1983)).

The expression cassette comprising the structural gene for CMIII operably linked to the desired control sequences can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells. Typically, genes conferring resistance to antibiotics or selected herbicides are used. After the genetic material is introduced into the target cells, successfully transformed cells and/or colonies of cells can be isolated by selection on the basis of these markers.

Typically, an intermediate host cell will be used in the practice of this invention to increase the copy number of the cloning vector. With an increased copy number, the vector containing the gene of interest can be isolated in significant quantities for introduction into the desired plant cells. Host cells that can be used in the practice of this invention include prokaryotes, including bacterial hosts such as E. coli, S. typhimurium, and Serratia marcescens. Eukaryotic hosts such as yeast or filamentous fungi may also be used in this invention. Since these hosts are also microorganisms, it will be essential to ensure that plant promoters which do not cause expression of the CMIII in bacteria are used in the vector.

The isolated cloning vector will then be introduced into the plant cell using any convenient technique, including electroporation (in protoplasts), retroviruses, bombardment, and microinjection, into cells from monocotyledonous or dicotyledonous plants, in cell or tissue culture, to provide transformed plant cells containing as foreign DNA at least one copy of the DNA sequence of the plant expression cassette. Preferably, the monocotyledonous species will be selected from maize, sorghum, wheat and rice, and the dicotyledonous species will be selected from soybean, alfalfa, tobacco and tomato. Using known techniques, protoplasts can be regenerated and cell or tissue culture can be regenerated to form whole fertile plants which carry and express the gene for CMIII. Accordingly, a highly preferred embodiment of the present invention is a transformed maize plant, the cells of which contain as foreign DNA at least one copy of the DNA sequence of an expression cassette of this invention.

Finally, this invention provides methods of imparting resistance to diseases caused by microorganisms selected from Fusarium graminearum, Fusarium moniliforme, Aspergillus flavus, Alternaria longipes, Sclerotinia sclerotiorum, and Sclerotinia trifoliorum to plants of a susceptible taxon, comprising the steps of:
a) culturing cells or tissues from at least one plant from the taxon,
b) introducing into the cells of the cell or tissue culture at least one copy of an expression cassette comprising a structural gene for CMIII, operably linked to plant regulatory sequences which cause the expression of the CMIII structural gene in the cells, and
c) regenerating disease-resistant whole plants from the cell or tissue culture. Once whole plants have been obtained, they can be sexually or clonally reproduced in such manner that at least one copy of the sequence provided by the expression cassette is present in the cells of progeny of the reproduction.

Alternatively, once a single transformed plant has been obtained by the foregoing recombinant DNA method, conventional plant breeding methods can be used to transfer the CMIII structural gene and associated regulatory sequences via crossing and backcrossing. Such intermediate methods will comprise the further steps of
a) sexually crossing the disease-resistant plant with a plant from the disease-susceptible taxon;
b) recovering reproductive material from the progeny of the cross; and
c) growing disease-resistant plants from the reproductive material. Where desirable or necessary, the agronomic characteristics of the susceptible taxon can be substantially preserved by expanding this method to include the further steps of repetitively:

a) backcrossing the disease-resistant progeny with disease-susceptible plants from the susceptible taxon; and
b) selecting for expression of antimicrobial activity (or an associated marker gene) among the progeny of the backcross, until the desired percentage of the characteristics of the susceptible taxon are present in the progeny along with the gene imparting antimicrobial activity.

By the term "taxon" herein is meant a unit of botanical classification of genus or lower. It thus includes genus, species, cultivars, varieties, variants, and other minor taxonomic groups which lack a consistent nomenclature.

It will also be appreciated by those of ordinary skill that the plant vectors provided herein can be incoporated into Agrobacterium tumefaciens, which can then be used to transfer the vector into susceptible plant cells, primarily from dicotyledonous species. Thus, this invention provides a method for imparting antimicrobial activity and disease resistance in Agrobacterium tumefaciens-susceptible dicotyledonous plants in which the expression cassette is introduced into the cells by infecting the cells with Agrobacterium tumefaciens, a plasmid of which has been modified to include a plant expression cassette of this invention.

The following description further exemplifies the compositions of this invention and the methods of makinq and using them. However, it will be understood that other methods, known by those of ordinary skill in the art to be equivalent, can also be employed.

### Example 1

### Purification of CMIII.

CMIII was obtained from two sources, the public corn variety B73 and proprietary corn variety MH18 as follows:

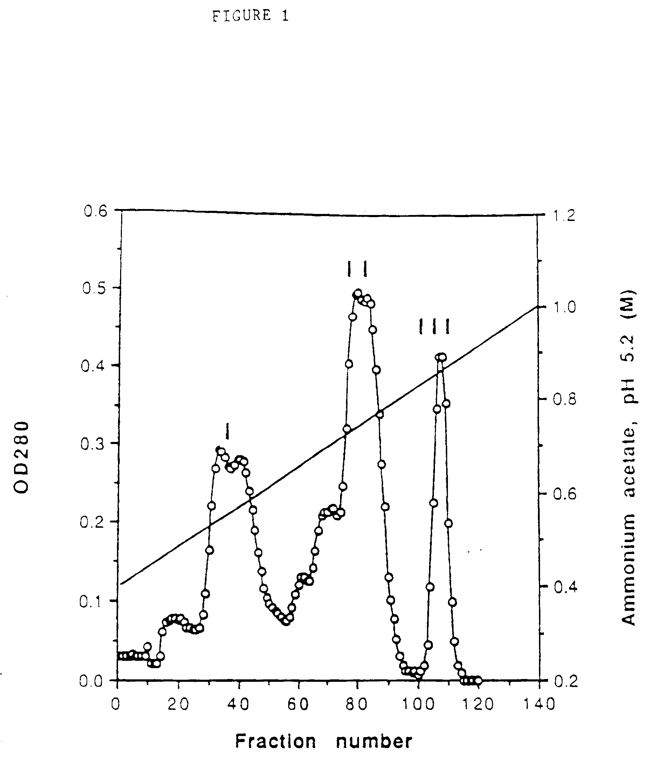

Ground maize seeds were extracted at room temperature with 0.1 N H₂SO₄. The resulting extract was neutralized and centrifuged to remove insoluble material. The extract was adjusted to pH 5.2 and chromatographed on a carboxymethyl cellulose cation exchange column equilibrated in 0.3 M ammonium acetate, eluting with a gradient of ammonium acetate of from 0.4 to 1.0 M. (Figure 1). CMIII eluted at approximately 0.8 M in a series of A280 peaks that also includes other peptides of similar size and properties.

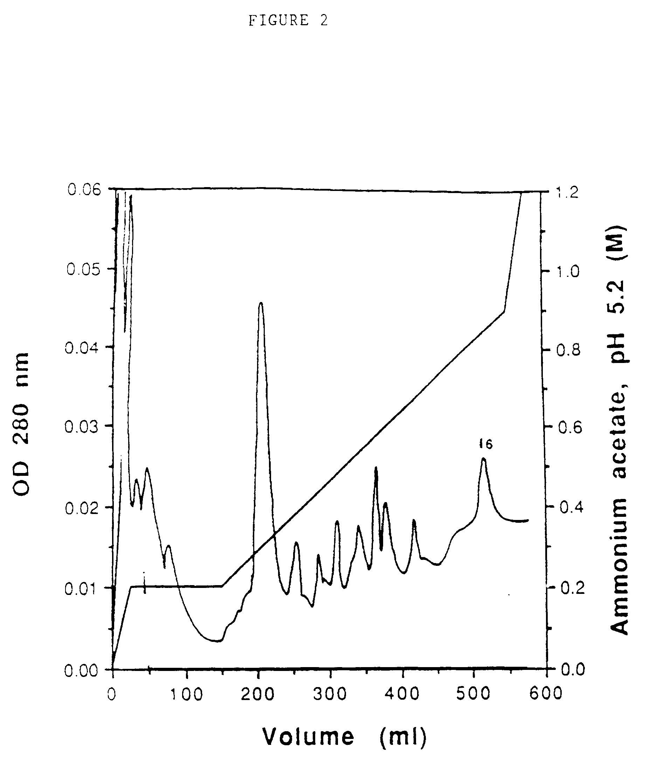

CMIII can also be purified by chromatography on a Mono-S cation exchange FPLC column (Pharmacia) with a similar gradient of ammonium acetate. Peak MS-16 corresponds to CMIII in Figure 2.

CMIII elutes as a single major UV-absorbing peak from a reverse phase HPLC column (C₁₈, equilibrated in 0.1% TFA; 10-40% gradient of acetonitrile/0.1% TFA), indicating that it is substantially free of contaminating proteins or peptides.

### Example 2

### Characterization of CMIII

CMIII migrated as a single coomassie blue-staining band in both SDS-urea and SDS-TRIS-Tricine polyacrylamide gel systems, with a relative mobility indicating a molecular weight of 4000 daltons. Amino acid analysis showed a high proportion of arginine (10 residues/mole), glutamate/glutamine (7 residues/mole) and cysteine (4 residues/mole).

Tryptophan, which is not detected by amino acid analysis, was detected (approx. 1 residue/mole) by quantitative oxidation with N-bromosuccinimide. Circular dichroism spectroscopy indicates a high alpha-helical content.

The first 24 N-terminal amino acids of CMIII were identified by sequencing of peptide blotted from TRIS-Tricine SDS-PAGE gels to Immobilon P polyvinylidine fluoride membranes. In addition, five endo-Gluc-C fragments of CMIII were purified by reverse phase HPLC and sequenced, confirming and tentatively extending the peptide sequence to a total of 34 residues, for a total molecular weight of approximately 3900 daltons. In this way, CMIII was found to have the sequence Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly.

From this analysis, it can be seen that CMIII is structurally different from any of the monocot thionins, which unlike CMIII are high in lysine and low in arginine. CMIII is surprisingly similar to a mammalian antimicrobial peptide, defensin NP1 from rabbits, although CMIII is much higher in glutamate and has 4 rather than 6 cysteines. There is slight similarity to a basic peptide obtained from pumpkin seeds (Naisbitt et al., Plant Physiol. 1988, 88:770) which is high in arginine and glutamate/glutamine but which lacks cysteine.

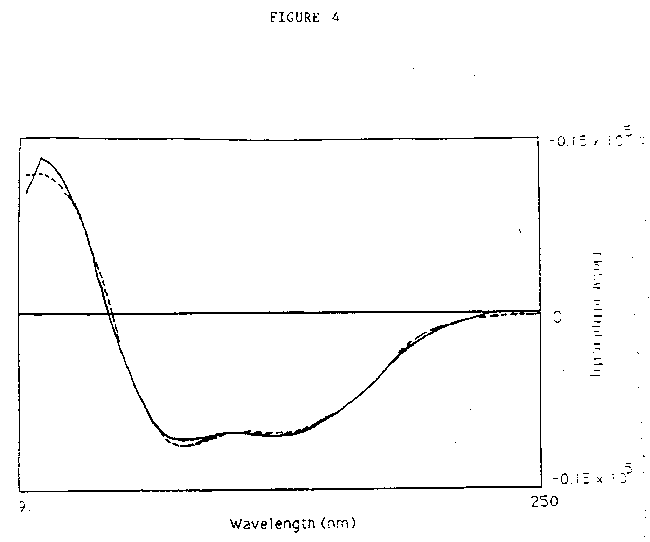

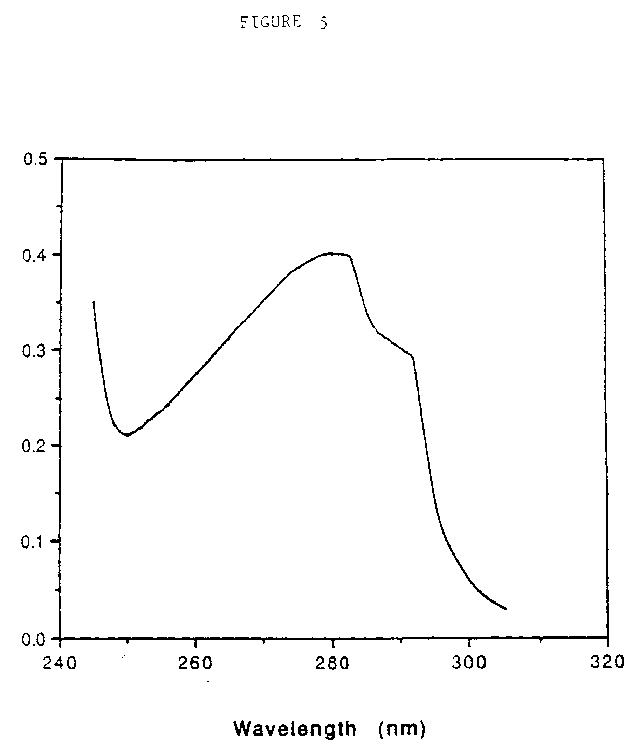

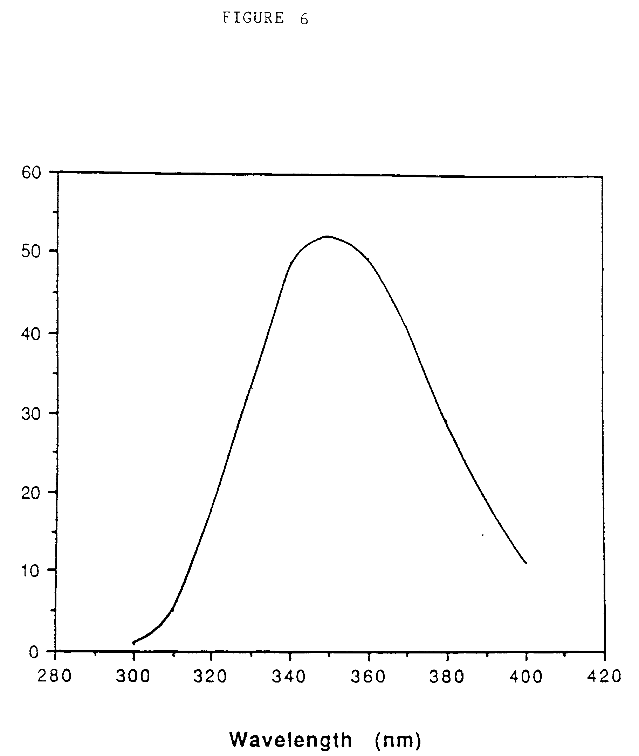

CM-III was further characterized by its CD, UV-absorption and flourescence spectra, as illustrated in Figures 4, 5 & 6.

### Example 3

### Identification of the CMIII gene and insertion into bacteria

The amino acid sequence of CMIII includes several stretches that are amenable to construction of synthetic, degenerate oligonucleotide probes; one example is residues 20-26 (Try-Glu-Thr-Gln-Glu-Cys-Met-Arg), which is coded by the nucleotide sequence TGG GAR ACN CAR GAR TGY ATG MG, where Y = T or C, R = A or G, M = A or C, and N = A or G or T or C. Another, overlapping stretch is residues 15-21 (His-Glu-Gly-Gln-Pro-Tryp-Glu-Thr), which can be coded for by the following : CAY GAR GGN CAR CCN TGG GAR AC. These oligonucleotides are end-labelled with ³²p and used to screen a maize genomic or cDNA library. Clones which hybridize to both probes are subcloned into an appropriate single-stranded phage vector such as Bluescript KS+, restriction sites in the cloned DNA are mapped, and the region corresponding to the CMIII gene identified by Southern hybridization of restriction fragments using the oligonucleotides as probes. The CMIII gene region is subcloned and sequenced to verify identity and determine the open reading frame for protein synthesis. Finally, restriction sites are engineered at either end of the open reading frame by site-directed mutagenesis, to allow the gene to be introduced into a plant expression cassette.

Alternatively, of course, a complete synthetic gene can be constructed from the sequence given herein for the CMIII protein.

### Example 4

### Expression of the CMIII Gene in Plants

A plant expression cassette, employing the regulatory sequences developed by Beach, et al., and containing the CMIII gene, is constructed. The preferred plasmid, designated pPHI414, contains an enhanced 35S promoter spanning nucleotides - 421 to +2 of Cauliflower Mosaic Virus with the region form - 421 to - 90 duplicated in tandem, a 79 bp Sall HindIII fragment from pJII101 spanning the 5′ leader sequence of Tobacco Mosaic Virus, a 579 bp fragment spanning the first intron from maize AdH1-S, and a 281 bp fragment spanning the polyadenylation site from the nopaline synthase gene in pTiT37.

Another construct which can be used as an expression cassette is the pPHI412 plasmid . It differs from pPHI414 in that it lacks the AdH intron segment. However, like pPHI414, it is constructed to have numerous restriction sites between the O′ segment and the NOS segment, which sites can be conveniently used for splicing the CMIII structural gene into position.

This vector can be cotransformed with a similar plasmid containing a selectable marker for antibiotic resistance into Black Mexican Sweet corn protoplasts by electroporation. These protoplasts can then be induced to regenerate cell walls and develop into callus by conventional techniques. Likewise, this callus can then be subjected to antibiotic selection to select for transformed colonies, and these colonies can be tested for expression of CMIII with antisera for the appropriate CMIII using known methods. The efficiency of protection can be measured by infesting callus (or suspension cultures derived from callus) with the target microorganism and measuring survival percentages.

The CMIII gene can be introduced into embryogenic maize callus by methods similar to those used for Black Mexican Sweet. Embryogenic callus can be regenerated to whole fertile plants. The antimicrobial activity and disease resistance imparted by the endogenous production of the CMIII is a simply inherited, dominant trait and can, if desired, be introduced into other plant varieties of the species by simple crossing or backcrossing.

As used herein, the term "antimicrobial amount" means at least an LD₅₀ of the CMIII as measured against a target organism.

### Example 5

### Microorganism Susceptibility to CMIII

In spore germination and hyphal growth assays against plant pathogenic fungi, CMIII has been found to have inhibitory activity against Fusarium graminearum, Sclerotinia sclerotiorum, and Sclerotinia trifoliorum at a concentration of 112 µg/mL. Other fungi, including Fusarium moniliforme and Aspergillus flavus, were slightly inhibited at that concentration. CMIII activity is reduced substantially in the presence of plant cell extracts.

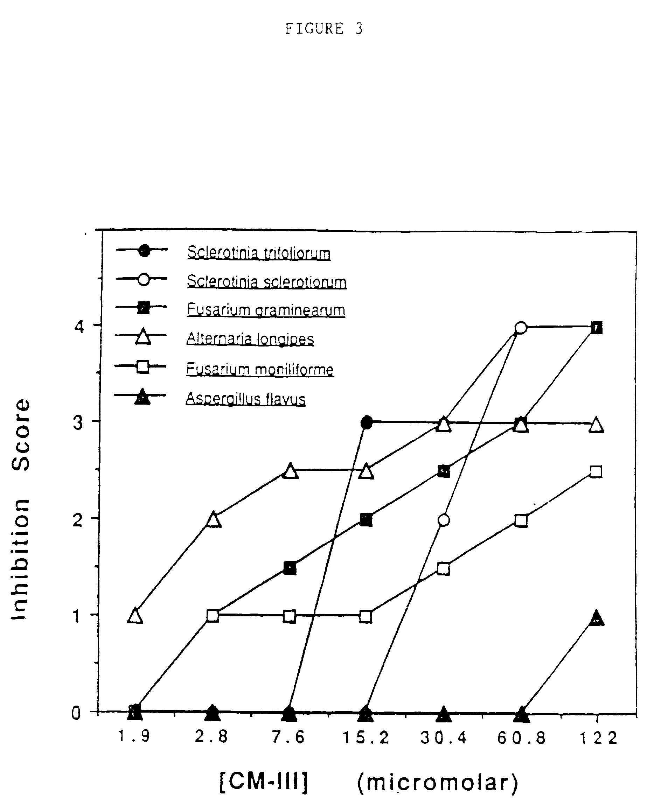

Approximately 150 conidia or approximately 5 to 20 mycelial fragments were incubated in 90 microliters of culture medium in microtiter plate wells. The culture medium was a broth medium containing 0.25% yeast extract, 0.1% casein hydrolyzate, 1% glucose, 0.025% calcium nitrate, 0.005% potassium phosphate, and 0.00625% magnesium sulfate. The peptide at the test concentration was added in 10 microliters in water or buffer, and the plates were incubated from 1 to three days at 28 degrees C. At the end of the incubation period, the wells were examined and mycelial growth was scored versus control wells as follows:
0 = No inhibition
1 = Slight Inhibition
2 = Moderate Inhibition
3 = Almost Complete Inhibition
4 = Complete Inhibition
results of one such study are indicated in Figure 3.

In other studies, Alternaria longipes was found to be the most sensitive of the fungi to the action of CMIII, showing noticeable inhibition at 1.9 micromolar. Aspergillus flavus was the most resistant, showing slight inhibition at 122 micromolar. The maize seed pathogens Fusarium graminearum and Fusarium moniliforme were noticeably inhibited down to 2.8 micromolar, although F. moniliforme was not completely inhibited even at 122 micromolar. Sclerotinia isolates were inhibited strongly at higher concentrations, but inhibition was not observed at lower concentrations (below 30.4 micromolar for S. sclerotiorum, and below 15.2 micromolar for S. trifoliorum.)

In yet another study A. longipes was not affected by CMIII at 3 µM.

In summary, CMIII is inhibitory to a wide range of plant pathogens, although inhibition profiles vary widely from pathogen to pathogen.

## Claims (Claims for the following Contracting State(s): AT, BE, CH, LI, DE, DK, FR, GB, GR, IT, LU, NL, SE)

1. A method for killing or inhibiting a plant pathogenic microorganism which is susceptible to the antimicrobial polypeptide CMIII having the amino acid sequence Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly, comprising providing plants having inserted into their genome a DNA sequence coding for CMIII, in proper reading frame relative to transcription initiator and promoter sequences active in the plant, so as to cause expression of an antimicrobial amount of CMIII in tissues of the plant which are normally infected by said pathogen.

2. A method according to claim 1 wherein said microorganism is *Fusarium graminearum*, *Fusarium moniliforme*, *Aspergillus flavus*, *Alternaria longipes*, *Sclerotinia sclerotiorum*, or *Sclerotinia trifoliorum*.

3. A method according to claim 1 or claim 2 wherein said plants are of a monocotyledonous species selected from wheat, rice and sorghum.

4. A method according to any one of claims 1 to 3 further comprising the steps of:
a) culturing cells or tissues from a plant,
b) introducing into the cells of the cell or tissue culture at least one copy of an expression cassette comprising a sequence coding for CMIII, and
c) regenerating protected whole plants from the cell or tissue culture.

5. A method according to claim 4 which comprises the further step of sexually or clonally reproducing the whole plant in such manner that at least one copy of the sequence provided by the expression cassette is present in the cells of progeny of the reproduction.

6. A method according to claim 4 or claim 5 in which the expression cassette is introduced into the cells by electroporation.

7. A method according to claim 4 or claim 5 in which the expression cassette is introduced into the cells by microparticle bombardment.

8. A method according to claim 4 or claim 5 in which the expression cassette is introduced into the cells by microinjection.

9. A method according to claim 4 or claim 5 for providing resistance to microorganisms in *Agrobacterium tumefaciens*-susceptible dicotyledonous plants in which the expression cassette is introduced into the cells by infecting the cells with *Agrobacterium tumefaciens*, a plasmid of which has been modified to include the expression cassette.

10. A method of imparting resistance to disease caused by plant pathogens selected from *Fusarium graminearum*, *Fusarium moniliforme*, *Aspergillus flavus*, *Alternaria longipes*, *Sclerotinia sclerotiorium* and *Sclerotinia trifoliorum*, to plants of a taxon susceptible to those diseases, comprising the steps of:
a) selecting a fertile, disease resistant plant prepared by a method of claim 4 from a sexually compatible plant;
b) sexually crossing the disease resistant plant with a plant from the disease susceptible taxon;
c) recovering reproductive material from the progeny of the cross; and
d) growing protected plants from the reproductive material.

11. A method according to claim 10 for imparting disease resistance and antimicrobial activity in a taxon consisting of substantially homozygous plants, which comprises the further steps of repetitively:
a) backcrossing the disease resistant progeny with substantially homozygous, disease susceptible plants from the taxon; and
b) selecting for expression of disease resistance and antimicrobial activity along with the other desired characteristics of the susceptible taxon from among the progeny of the backcross, until the desired percentage of the characteristics of the susceptible taxon are present in the progeny along with disease resistance and antimicrobial activity.

12. A recombinant DNA which codes substantially solely for CMIII having the amino acid sequence Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly.

13. A recombinant DNA as claimed in claim 12 which comprises an expression cassette wherein the coding sequence for CMIII is operably linked to bacterial expression regulatory sequences which cause the expression of CMIII in bacterial cells.

14. Bacterial cells containing as a foreign plasmid at least one copy of an expression cassette according to claim 13.

15. A recombinant DNA as claimed in claim 12 which comprises an expression cassette wherein the coding sequence for CMIII is operably linked to plant regulatory sequences which cause the expression of CMIII in plant cells.

16. Transformed plant cells containing as foreign DNA at least one copy of the DNA sequence of an expression cassette according to claim 15.

17. Transformed cells according to claim 16 which are cells of a monocotyledonous species.

18. Transformed cells according to claim 17, which are maize, sorghum, wheat or rice cells.

19. Transformed cells according to claim 16, which are cells of a dicotyledonous species.

20. Transformed cells according to claim 19, which are soybean, alfalfa, tobacco or tomato cells.

21. A maize cell or tissue culture comprising cells according to claim 18.

22. A transformed plant, the cells of which contain as foreign DNA at least one copy of the DNA sequence of an expression cassette according to claim 15.

23. A transformed plant obtainable by a method according to any one of claims 4 to 11.

24. A transformed plant according to claim 22 or 23 which is of a species as defined in any one of claims 17 to 20.

25. A transformed plant according to claim 24 which is a maize plant.

26. A transformed plant according to any one of claims 22 to 25 which is not a plant variety.

## Claims (Claims for the following Contracting State(s): ES)

1. A method for killing or inhibiting a plant pathogenic microorganism which is susceptible to the antimicrobial polypeptide CMIII having the amino acid sequence Ag-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly, comprising providing plants having inserted into their genome a DNA sequence coding for CMIII, in proper reading frame relative to transcription initiator and promoter sequences active in the plant, so as to cause expression of an antimicrobial amount of CMIII in tissues of the plant which are normally infected by said pathogen.

2. A method according to claim 1 wherein said microorganism is *Fusarium graminearum*, *Fusarium moniliforme*, *Aspergillus flavus*, *Alternaria longipes*, *Sclerotinia sclerotiorum*, or *Sclerotinia trifoliorum*.

3. A method according to claim 1 or claim 2 wherein said plants are of a monocotyledonous species selected from wheat, rice and sorghum.

4. A method according to any one of claims 1 to 3 further comprising the steps of:
a) culturing cells or tissues from a plant,
b) introducing into the cells of the cell or tissue culture at least one copy of an expression cassette comprising a sequence coding for CMIII, and
c) regenerating protected whole plants from the cell or tissue culture.

5. A method according to claim 4 which comprises the further step of sexually or clonally reproducing the whole plant in such manner that at least one copy of the sequence provided by the expression cassette is present in the cells of progeny of the reproduction.

6. A method according to claim 4 or claim 5 in which the expression cassette is introduced into the cells by electroporation.

7. A method according to claim 4 or claim 5 in which the expression cassette is introduced into the cells by microparticle bombardment.

8. A method according to claim 4 or claim 5 in which the expression cassette is introduced into the cells by microinjection.

9. A method according to claim 4 or claim 5 for providing resistance to microorganisms in *Agrobacterium tumefaciens*-susceptible dicotyledonous plants in which the expression cassette is introduced into the cells by infecting the cells with *Agrobacterium tumefaciens*, a plasmid of which has been modified to include the expression cassette.

10. A method of imparting resistance to disease caused by plant pathogens selected from *Fusarium graminearum*, *Fusarium moniliforme*, *Aspergillus flavus*, *Alternaria longipes*, *Sclerotinia sclerotiorium* and *Sclerotinia trifoliorum*, to plants of a taxon susceptible to those diseases, comprising the steps of:
a) selecting a fertile, disease resistant plant prepared by a method of claim 4 from a sexually compatible plant;
b) sexually crossing the disease resistant plant with a plant from the disease susceptible taxon;
c) recovering reproductive material from the progeny of the cross; and
d) growing protected plants from the reproductive material.

11. A method according to claim 10 for imparting disease resistance and antimicrobial activity in a taxon consisting of substantially homozygous plants, which comprises the further steps of repetitively:
a) backcrossing the disease resistant progeny with substantially homozygous, disease susceptible plants from the taxon; and
b) selecting for expression of disease resistance and antimicrobial activity along with the other desired characteristics of the susceptible taxon from among the progeny of the backcross, until the desired percentage of the characteristics of the susceptible taxon are present in the progeny along with disease resistance and antimicrobial activity.

12. A method of producing a transformed bacterial cell containing as foreign DNA at least one copy of an expression cassette comprising a recombinant DNA which codes substantially solely for CMIII having the amino acid sequence Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly, which method comprises introducing said expression cassette into said cell.

13. A method of producing a transformed plant cell containing as foreign DNA at least one copy of an expression cassette comprising a recombinant DNA which codes substantially solely for CMIII having the amino acid sequence Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly wherein the coding sequence for CMIII is operably linked to plant regulatory sequences which cause the expression of CMIII in plant cells.

14. A method according to claim 13 wherein the cells are of a monocotyledonous species.

15. A method according to claim 14 wherein the cells are maize, sorghum, wheat or rice cells.

16. A method according to claim 13 wherein the cells are of a dicotyledonous species.

17. A method according to claim 16 wherein the cells are soybean, alfalfa, tobacco or tomato cells.

18. A recombinant DNA which codes substantially solely for CMIII having the amino acid sequence Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly.

19. A recombinant DNA as claimed in claim 18 which comprises an expression cassette wherein the coding sequence for CMIII is operably linked to bacterial expression regulatory sequences which cause the expression of CMIII in bacterial cells.

20. Bacterial cells containing as a foreign plasmid at least one copy of an expression cassette according to claim 19.

21. A recombinant DNA as claimed in claim 18 which comprises an expression cassette wherein the coding sequence of CMIII is operably linked to plant regulatory sequences which cause the expression of CMIII in plant cells.

22. Transformed plant cells containing as foreign DNA at least one copy of the DNA sequence of an expression cassette according to claim 21.

23. Transformed cells according to claim 22 which are cells of a monocotyledonous species.

24. Transformed cells according to claim 23, which are maize, sorghum, wheat or rice cells.

25. Transformed cells according to claim 22, which are cell of a dicotyledonous species.

26. Transformed cells according to claim 25, which are soybean, alfalfa, tobacco or tomato cells.

27. A maize cell or tissue culture comprising cells according to claim 24.

28. A transformed plant, the cells of which contain as foreign DNA at least one copy of the DNA sequence of an expression cassette according to claim 21.

29. A transformed plant obtainable by a method according to any one of claims 4 to 11.

30. A transformed plant according to claim 28 or 29 which is of a species as defined in any one of claims 23 to 26.

31. A transformed plant according to claim 30 which is a maize plant.

32. A transformed plant according to any one of claims 28 to 31 which is not a plant variety.

## Patentansprüche (Patentansprüche für folgende(n) Vertragsstaat(en): AT, BE, CH, LI, DE, DK, FR, GB, GR, IT, LU, NL, SE)

1. Verfahren zum Abtöten oder Inhibieren eines pflanzenpathogenen Mikroorganismus, welcher gegenüber dem antimikrobiellen Polypeptid CMIII mit der Aminosäuresequenz Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly empfindlich ist, umfassend das Zurverfügungstellen von Pflanzen, die in ihrem Genom eine DNA-Sequenz insertiert haben, die für CMIII codiert, im richtigen Leserahmen bezogen auf Transskriptions-, Initiations- und Promotorsequenzen, die in der Pflanze aktiv sind, so daß die Expression einer antimikrobiellen Menge von CMIII in Geweben der Pflanze bewirkt wird, welche normalerweise durch dieses Pathogen infiziert werden.

2. Verfahren nach Anspruch 1, wobei der besagte Mikroorganismus Fusarium graminearum, Fusarium moniliforme, Aspergillus flavus, Alternaria longipes, Sclerotinia sclerotiorum oder Sclerotinia trifoliorum ist.

3. Verfahren nach Anspruch 1 oder 2, wobei diese Pflanzen monocotyledone Arten sind, ausgewählt aus Weizen, Reis und Sorghum.

4. Verfahren nach einem der Ansprüche 1 bis 3, weiterhin umfassend die Stufen:
a) Kultivieren von Zellen oder Geweben aus einer Pflanze,
b) Einführen in die Zellen der Zell- oder Gewebekultur von mindestens einer Kopie einer Expressionskassette, umfassend eine Sequenz, die für CMIII codiert, und
c) Regenerieren geschützter vollständiger Pflanzen aus der Zell- oder Gewebekultur.

5. Verfahren nach Anspruch 4, welches den weiteren Schritt des sexuellen oder klonalen Vermehrens der vollständigen Pflanze in einer solchen Weise umfaßt, daß mindestens eine Kopie der durch die Expressionskassette bereitgestellten Sequenz in den Zellen der Nachkommenschaft der Vermehrung vorhanden ist.

6. Verfahren nach Anspruch 4 oder Anspruch 5, bei dem die Expressionskassette in die Zellen durch Elektroporation eingeführt wird.

7. Verfahren nach Anspruch 4 oder Anspruch 5, bei dem die Expressionskassette in die Zellen durch Mikropartikelbombardierung eingeführt wird.

8. Verfahren nach Anspruch 4 oder Anspruch 5, bei dem die Expressionskassette in die Zellen durch Mikroinjektion eingeführt wird.

9. Verfahren nach Anspruch 4 oder Anspruch 5 zur Verleihung von Resistenz gegen Mikroorganismen bei Agrobacterium-tumefaciens-anfälligen dicotyledonen Pflanzen, bei dem die Expressionskassette in die Zellen durch Infizieren der Zellen mit Agrobacterium tumefaciens, bei dem ein Plasmid so modifiziert wurde, daß es die Expressionskassette beinhaltet, eingeführt wird.

10. Verfahren zum Übertragen von Resistenz gegen Krankheiten, die durch Pflanzenpathogene ausgewählt aus Fusarium graminearum, Fusarjum moniliforme, Aspergillus flavus, Alternaria longipes, Sclerotinia sclerotiorium und Sclerotinia trifoliorum bewirkt werden, auf Pflanzen eines Taxons, welche anfällig gegenüber diesen Krankheiten sind, umfassend die Stufen:
a) Auswählen einer fruchtbaren, krankheitsresistenten Pflanze, hergestellt aus einer sexuell kompatiblen Pflanze nach einem Verfahren nach Anspruch 4;
b) sexuelles Kreuzen der krankheitsresistenten Pflanze mit einer Pflanze aus dem krankheitsempfänglichen Taxon;
c) Gewinnen von fortpflanzungsfähigem Material aus der Nachkommenschaft der Kreuzung; und
d) Züchten geschützter Pflanzen aus dem fortpflanzungsfähigen Material.

11. Verfahren nach Anspruch 10 zur Übertragung von Krankheitsresistenz und antimikrobieller Aktivität auf ein Taxon, welches aus im wesentlichen homozygoten Pflanzen besteht, umfassend die weiteren wiederholten Stufen:
a) Rückkreuzen der krankheitsresistenten Nachkommenschaft mit im wesentlichen homozygoten, krankheitsanfälligen Pflanzen des Taxons; und
b) Selektionieren auf eine Ausprägung der Krankheitsresistenz und eine antimikrobielle Aktivität hin, zusammen mit den anderen erwünschten Eigenschaften des anfälligen Taxons aus der Nachkommenschaft der Rückkreuzung, bis der gewünschte Prozentsatz der Eigenschaften des anfälligen Taxons in der Nachkommenschaft zusammen mit Krankheitsresistenz und antimikrobieller Aktivität vorhanden sind.

12. Rekominante DNA, welche im wesentlichen ausschließlich für CMIII codiert, welches die Aminosäuresequenz Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly aufweist.

13. Rekombinante DNA nach Anspruch 12, welche eine Expressionskassette umfaßt, worin die für CMIII codierende Sequenz funktionsfähig verknüpft ist mit bakteriellen expressionsregulatorischen Sequenzen, die die Expression von CMIII in bakteriellen Zellen bewirken.

14. Bakterielle Zellen, enthaltend als ein fremdes Plasmid mindestens eine Kopie einer Expressionskassette nach Anspruch 13.

15. Rekombinante DNA nach Anspruch 12, welche eine Expressionskassette umfaßt, worin die für CMIII codierende Sequenz funktionsfähig verknüpft ist mit pflanzenregulatorischen Sequenzen, welche die Expression von CMIII in Pflanzenzellen bewirken.

16. Transformierte Pflanzenzellen, enthaltend als Fremd-DNA mindestens eine Kopie der DNA-Sequenz einer Expressionskassette nach Anspruch 15.

17. Transformierte Zellen nach Anspruch 16, welche Zellen einer monocotyledonen Art sind.

18. Transformierte Zellen nach Anspruch 17, welche Mais-, Sorghum-, Weizen- oder Reiszellen sind.

19. Transformierte Zellen nach Anspruch 16, welche Zellen einer dicotyledonen Art sind.

20. Transformierte Zellen nach Anspruch 19, welche Sojabohnen-, Alfalfa-, Tabak- oder Tomatenzellen sind.

21. Eine Maiszellen- oder Maisgewebekultur, umfassend Zellen nach Anspruch 18.

22. Transformierte Pflanze, deren Zellen als Fremd-DNA mindestens eine Kopie der DNA-Sequenz einer Expressionskassette nach Anspruch 15 enthalten.

23. Transformierte Pflanze, erhältlich nach einem Verfahren nach einem der Ansprüche 4 bis 11.

24. Transformierte Pflanze nach Anspruch 22 oder 23, welche aus einer in einem der Ansprüche 17 bis 20 definierten Art stammt.

25. Transformierte Pflanze nach Anspruch 24, welche eine Maispflanze ist.

26. Transformierte Pflanze nach einem der Ansprüche 22 bis 25, welche nicht eine Pflanzenvarietät ist.

## Patentansprüche (Patentansprüche für folgende(n) Vertragsstaat(en): ES)

1. Verfahren zum Abtöten oder Inhibieren eines pflanzenpathogenen Mikroorganismus, welcher gegenüber dem antimikrobiellen Polypeptid CMIII mit der Aminosäuresequenz Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly empfindlich ist, umfassend das Zurverfügungstellen von Pflanzen, die in ihrem Genom eine DNA-Sequenz insertiert haben, die für CMIII codiert, im richtigen Leserahmen bezogen auf Transskriptions-, Initiations- und Promotorsequenzen, die in der Pflanze aktiv sind, so daß die Expression einer antimikrobiellen Menge von CMIII in Geweben der Pflanze bewirkt wird, welche normalerweise durch dieses Pathogen infiziert werden.

2. Verfahren nach Anspruch 1, wobei der besagte Mikroorganismus Fusarium graminearum, Fusarium moniliforme, Aspergillus flavus, Alternaria longipes, Sclerotinia sclerotiorum oder Sclerotinia trifoliorum ist.

3. Verfahren nach Anspruch 1 oder 2, wobei diese Pflanzen monocotyledone Arten sind, ausgewählt aus Weizen, Reis und Sorghum.

4. Verfahren nach einem der Ansprüche 1 bis 3, weiterhin umfassend die Stufen:
a) Kultivieren von Zellen oder Geweben aus einer Pflanze,
b) Einführen in die Zellen der Zell- oder Gewebekultur von mindestens einer Kopie einer Expressionskassette, umfassend eine Sequenz, die für CMIII codiert, und
c) Regenerieren geschützter vollständiger Pflanzen aus der Zell- oder Gewebekultur.

5. Verfahren nach Anspruch 4, welches den weiteren Schritt des sexuellen oder klonalen Vermehrens der vollständigen Pflanze in einer solchen Weise umfaßt, daß mindestens eine Kopie der durch die Expressionskassette bereitgestellten Sequenz in den Zellen der Nachkommenschaft der Vermehrung vorhanden ist.

6. Verfahren nach Anspruch 4 oder Anspruch 5, bei dem die Expressionskassette in die Zellen durch Elektroporation eingeführt wird.

7. Verfahren nach Anspruch 4 oder Anspruch 5, bei dem die Expressionskassette in die Zellen durch Mikropartikelbombardierung eingeführt wird.

8. Verfahren nach Anspruch 4 oder Anspruch 5, bei dem die Expressionskassette in die Zellen durch Mikroinjektion eingeführt wird.

9. Verfahren nach Anspruch 4 oder Anspruch 5 zur Verleihung von Resistenz gegen Mikroorganismen bei Agrobacterium-tumefaciens-anfälligen dicotyledonen Pflanzen, bei dem die Expressionskassette in die Zellen durch Infizieren der Zellen mit Agrobacterium tumefaciens, bei dem ein Plasmid so modifiziert wurde, daß es die Expressionskassette beinhaltet, eingeführt wird.

10. Verfahren zum Übertragen von Resistenz gegen Krankheiten, die durch Pflanzenpathogene ausgewählt aus Fusarjum graminearum, Fusarjum moniliforme, Aspergillus flavus, Alternaria longipes, Sclerotinia sclerotiorium und Sclerotinia trifoliorum bewirkt werden, auf Pflanzen eines Taxons, welche anfällig gegenüber diesen Krankheiten sind, umfassend die Stufen:
a) Auswählen einer fruchtbaren, krankheitsresistenten Pflanze, hergestellt aus einer sexuell kompatiblen Pflanze nach einem Verfahren nach Anspruch 4;
b) sexuelles Kreuzen der krankheitsresistenten Pflanze mit einer Pflanze aus dem krankheitsempfänglichen Taxon;
c) Gewinnen von fortpflanzungsfähigem Material aus der Nachkommenschaft der Kreuzung; und
d) Züchten geschützter Pflanzen aus dem fortpflanzungsfähigen Material.

11. Verfahren nach Anspruch 10 zur Übertragung von Krankheitsresistenz und antimikrobieller Aktivität auf ein Taxon, welches aus im wesentlichen homozygoten Pflanzen besteht, umfassend die weiteren wiederholten Stufen:
a) Rückkreuzen der krankheitsresistenten Nachkommenschaft mit im wesentlichen homozygoten, krankheitsanfälligen Pflanzen des Taxons; und
b) Selektionieren auf eine Ausprägung der Krankheitsresistenz und eine antimikrobielle Aktivität hin, zusammen mit den anderen erwünschten Eigenschaften des anfälligen Taxons aus der Nachkommenschaft der Rückkreuzung, bis der gewünschte Prozentsatz der Eigenschaften des anfälligen Taxons in der Nachkommenschaft zusammen mit Krankheitsresistenz und antimikrobieller Aktivität vorhanden sind.

12. Verfahren zur Herstellung einer transformierten Bakterienzelle, enthaltend als Fremd-DNA mindestens eine Kopie einer Expressionskassette, umfassend eine rekombinante DNA, welche im wesentlichen ausschließlich für CMIII codiert, welches die Aminosäuresequenz Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly aufweist, wobei das Verfahren das Einführen dieser Expressionskassette in diese Zelle umfaßt.

13. Verfahren zur Herstellung einer transformierten Pflanzenzelle, die als Fremd-DNA mindestens eine Kopie einer Expressionskassette enthält, welche eine rekombinante DNA umfaßt, welche im wesentlichen ausschließlich für CMIII codiert, welches die Aminosäuresequenz Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly aufweist, wobei die für CMIII codierende Sequenz funktionsfähig verbunden ist mit pflanzenregulatorischen Sequenzen, welche die Expression des CMIII in Pflanzenzellen bewirken.

14. Verfahren nach Anspruch 13, wobei die Zellen Zellen einer monocotyledonen Art sind.

15. Verfahren nach Anspruch 14, wobei die Zellen Mais-, Sorghum-, Weizen- oder Reiszellen sind.

16. Verfahren nach Anspruch 13, wobei die Zellen Zellen einer dicotyledonen Art sind.

17. Verfahren nach Anspruch 16, wobei die Zellen Sojabohnen-, Alfalfa-, Tabak- oder Tomatenzellen sind.

18. Rekominante DNA, welche im wesentlichen ausschließlich für CMIII codiert, welches die Aminosäuresequenz Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-cys-Arg-Arg-Arg-Gly Gly aufweist.

19. Rekombinante DNA nach Anspruch 18, welche eine Expressionskassette umfaßt, worin die für CMIII codierende Sequenz funktionsfähig verknüpft ist mit bakteriellen expressionsregulatorischen Sequenzen, die die Expression von CMIII in bakteriellen Zellen bewirken.

20. Bakterielle Zellen, enthaltend als ein fremdes Plasmid mindestens eine Kopie einer Expressionskassette nach Anspruch 19.

21. Rekombinante DNA nach Anspruch 18, welche eine Expressionskassette umfaßt, worin die für CMIII codierende Sequenz funktionsfähig verknüpft ist mit pflanzenregulatorischen Sequenzen, welche die Expression von CMIII in Pflanzenzellen bewirken.

22. Transformierte Pflanzenzellen, enthaltend als Fremd-DNA mindestens eine Kopie der DNA-Sequenz eine Expressionskassette nach Anspruch 21.

23. Transformierte Zellen nach Anspruch 22, welche Zellen einer monocotyledonen Art sind.

24. Transformierte Zellen nach Anspruch 23, welche Mais-, Sorghum-, Weizen- oder Reiszellen sind.

25. Transformierte Zellen nach Anspruch 22, welche Zellen einer dicotyledonen Art sind.

26. Transformierte Zellen nach Anspruch 25, welche Sojabohnen-, Alfalfa-, Tabak- oder Tomatenzellen sind.

27. Eine Maiszellen- oder Maisgewebekultur, umfassend Zellen nach Anspruch 24.

28. Transformierte Pflanze, deren Zellen als Fremd-DNA mindestens eine Kopie der DNA-Sequenz einer Expressionskassette nach Anspruch 21 enthalten.

29. Transformierte Pflanze, erhältlich nach einem Verfahren nach einem der Ansprüche 4 bis 11.

30. Transformierte Pflanze nach Anspruch 28 oder 29, welche aus einer in einem der Ansprüche 23 bis 26 definierten Art stammt.

31. Transformierte Pflanze nach Anspruch 30, welche eine Maispflanze ist.

32. Transformierte Pflanze nach einem der Ansprüche 28 bis 31, welche nicht eine Pflanzenvarietät ist.

## Revendications (Revendications pour l'(les) Etat(s) contractant(s) suivant(s): AT, BE, CH, LI, DE, DK, FR, GB, GR, IT, LU, NL, SE)

1. Méthode destinée à tuer ou à inhiber un micro-organisme pathogène de plante qui est sensible au polypeptide antimicrobien CMIII ayant la séquence d'acides aminés Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly, comprenant l'étape consistant à fournir des plantes ayant, insérée dans leur génome, une séquence d'ADN codant pour CMIII, dans un cadre de lecture approprié par rapport aux séquences d'initiation de la transcription et de promoteur actives dans la plante, de façon à induire l'expression d'une quantité antimicrobienne de CMIII dans des tissus de la plante qui sont normalement infectés par ledit agent pathogène.

2. Méthode selon la revendication 1 dans laquelle ledit micro-organisme est *Fusarium graminearum*, *Fusarium moniliforme*, *Aspergillus flavus*, *Alternaria longipes*, *Sclerotinia sclerotiorum*, ou *Sclerotinia trifoliorum*.

3. Méthode selon la revendication 1 ou la revendication 2 dans laquelle lesdites plantes sont d'une espèce monocotylédone choisie parmi le blé, le riz et le sorgho.

4. Méthode selon l'une quelconque des revendications 1 à 3 comprenant en outre les étapes consistant :
a) à cultiver des cellules ou des tissus d'une plante,
b) à introduire dans les cellules de la culture de cellules ou de tissu au moins une copie d'une cassette d'expression comprenant une séquence codant pour CMIII, et
c) à régénérer des plantes entières protégées à partir de la culture de cellules ou de tissu.

5. Méthode selon la revendication 4 qui comprend l'étape supplémentaire consistant à reproduire par voie sexuée ou clonale la plante entière de telle manière qu'au moins une copie de la séquence fournie par la cassette d'expression soit présente dans les cellules de la descendance de la reproduction.

6. Méthode selon la revendication 4 ou la revendication 5 dans laquelle la cassette d'expression est introduite dans les cellules par électroporation.

7. Méthode selon la revendication 4 ou la revendication 5 dans laquelle la cassette d'expression est introduite dans les cellules par bombardement de microparticules.

8. Méthode selon la revendication 4 ou la revendication 5 dans laquelle la cassette d'expression est introduite dans les cellules par microinjection.

9. Méthode selon la revendication 4 ou la revendication 5 destinée à conférer la résistance à des micro-organismes dans des plantes dicotylédones sensibles à *Agrobacterium tumefaciens* dans laquelle la cassette d'expression est introduite dans les cellules par infection des cellules avec *Agrobacterium tumefaciens*, dont un plasmide a été modifié pour inclure la cassette d'expression.

10. Méthode destinée à conférer la résistance à une maladie provoquée par des agents pathogènes de plante choisis parmi *Fusarium graminearum*, *Fusarium moniliforme*, *Aspergillus flavus*, *Alternaria longipes*, *Sclerotinia sclerotiorum*, et *Sclerotinia trifoliorum*, à des plantes d'un taxon sensible à ces maladies, comprenant les étapes consistant :
a) à choisir une plante fertile, résistante à la maladie, préparée par une méthode selon la revendication 4 à partir d'une plante sexuellement compatible ;
b) à effectuer un croisement sexué de la plante résistante à la maladie avec une plante du taxon sensible à la maladie ;
c) à récupérer le matériel reproducteur à partir de la descendance du croisement ; et
d) à faire croître des plantes protégées à partir du matériel reproducteur.

11. Méthode selon la revendication 10 destinée à conférer la résistance à une maladie et une activité antimicrobienne à un taxon essentiellement constitué de plantes homozygotes, qui comprend les étapes supplémentaires consistant, de manière répétée :
a) à effectuer un rétrocroisement (backcross) de la descendance résistante à la maladie avec des plantes essentiellement homozygotes, sensibles à la maladie du taxon, et
b) à sélectionner dans la descendance du rétrocroisement les plantes qui expriment la résistance à la maladie et l'activité antimicrobienne en même temps que les autres caractéristiques souhaitées du taxon sensible, jusqu'à ce que le pourcentage souhaité des caractéristiques du taxon sensible soit présent dans la descendance en même temps que la résistance à la maladie et l'activité antimicrobienne.

12. ADN recombiné qui code sensiblement uniquement pour CMIII ayant la séquence d'acides aminés Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly.

13. ADN recombiné tel que revendiqué à la revendication 12 qui comprend une cassette d'expression dans laquelle la séquence codante pour CMIII est liée de manière fonctionnelle à des séquences bactériennes régulatrices de l'expression qui induisent l'expression de CMIII dans des cellules bactériennes.

14. Cellules bactériennes contenant sous forme d'un plasmide étranger au moins une copie d'une cassette d'expression selon la revendication 13.

15. ADN recombiné tel que revendiqué à la revendication 12 qui comprend une cassette d'expression dans laquelle la séquence codante pour CMIII est liée de manière fonctionnelle à des séquences régulatrices de plante qui induisent l'expression de CMIII dans des cellules végétales.

16. Cellules de plante transformées contenant, sous forme d'ADN étranger, au moins une copie de la séquence d'ADN d'une cassette d'expression selon la revendication 15.

17. Cellules transformées selon la revendication 16 qui sont des cellules d'une espèce monocotylédone.

18. Cellules transformées selon la revendication 17, qui sont des cellules de maïs, de sorgho, de blé, ou de riz.

19. Cellules transformées selon la revendication 16, qui sont des cellules d'une espèce dicotylédone.

20. Cellules transformées selon la revendication 19, qui sont des cellules de soja, de luzerne, de tabac ou de tomate.

21. Culture de cellules ou de tissu de maïs comprenant des cellules selon la revendication 18.

22. Plante transformée, dont les cellules contiennent, sous forme d'ADN étranger, au moins une copie de la séquence d'ADN d'une cassette d'expression selon la revendication 15.

23. Plante transformée pouvant être obtenue par une méthode selon l'une quelconque des revendications 4 à 11.

24. Plante transformée selon la revendication 22 ou 23 qui est d'une espèce telle que définie dans l'une quelconque des revendications 17 à 20.

25. Plante transformée selon la revendication 24 qui est un plant de maïs.

26. Plante transformée selon l'une quelconque des revendications 22 à 25 qui n'est pas une variété de plante.

## Revendications (Revendications pour l'(les) Etat(s) contractant(s) suivant(s): ES)

1. Méthode destinée à tuer ou à inhiber un micro-organisme pathogène de plante qui est sensible au polypeptide antimicrobien CMIII ayant la séquence d'acides aminés Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly, comprenant l'étape consistant à fournir des plantes ayant, insérée dans leur génome, une séquence d'ADN codant pour CMIII, dans un cadre de lecture approprié par rapport aux séquences d'initiation de la transcription et de promoteur actives dans la plante, de façon à induire l'expression d'une quantité antimicrobienne de CMIII dans des tissus de la plante qui sont normalement infectés par ledit agent pathogène.

2. Méthode selon la revendication 1 dans laquelle ledit micro-organisme est *Fusarium graminearum*, *Fusarium moniliforme*, *Aspergillus flavus*, *Alternaria longipes*, *Sclerotinia sclerotiorum*, ou *Sclerotinia trifoliorum*.

3. Méthode selon la revendication 1 ou la revendication 2 dans laquelle lesdites plantes sont d'une espèce monocotylédone choisie parmi le blé, le riz et le sorgho.

4. Méthode selon l'une quelconque des revendications 1 à 3 comprenant en outre les étapes consistant :
a) à cultiver des cellules ou des tissus d'une plante,
b) à introduire dans les cellules de la culture de cellules ou de tissu au moins une copie d'une cassette d'expression comprenant une séquence codant pour CMIII, et
c) à régénérer des plantes entières protégées à partir de la culture de cellules ou de tissu.

5. Méthode selon la revendication 4 qui comprend l'étape supplémentaire consistant à reproduire par voie sexuée ou clonale la plante entière de telle manière qu'au moins une copie de la séquence fournie par la cassette d'expression soit présente dans les cellules de la descendance de la reproduction.

6. Méthode selon la revendication 4 ou la revendication 5 dans laquelle la cassette d'expression est introduite dans les cellules par électroporation.

7. Méthode selon la revendication 4 ou la revendication 5 dans laquelle la cassette d'expression est introduite dans les cellules par bombardement de microparticules.

8. Méthode selon la revendication 4 ou la revendication 5 dans laquelle la cassette d'expression est introduite dans les cellules par microinjection.

9. Méthode selon la revendication 4 ou la revendication 5 destinée à conférer la résistance à des micro-organismes dans des plantes dicotylédones sensibles à *Agrobacterium tumefaciens* dans laquelle la cassette d'expression est introduite dans les cellules par infection des cellules avec *Agrobacterium tumefaciens*, dont un plasmide a été modifié pour inclure la cassette d'expression.

10. Méthode destinée à conférer la résistance à une maladie provoquée par des agents pathogènes de plante choisis parmi *Fusarium graminearum*, *Fusarium monlilforme*, *Aspergillus flavus*, *Alternaria longipes*, *Sclerotinia sclerotiorum*, et *Sclerotinia trifoliorum*, à des plantes d'un taxon sensible à ces maladies, comprenant les étapes consistant :
a) à choisir une plante fertile, résistante à la maladie, préparée par une méthode selon la revendication 4 à partir d'une plante sexuellement compatible ;
b) à effectuer un croisement sexué de la plante résistante à la maladie avec une plante du taxon sensible à la maladie ;
c) à récupérer le matériel reproducteur à partir de la descendance du croisement ; et
d)à faire croître des plantes protégées à partir du matériel reproducteur.

11. Méthode selon la revendication 10 destinée à conférer la résistance à une maladie et une activité antimicrobienne à un taxon essentiellement constitué de plantes homozygotes, qui comprend les étapes supplémentaires consistant, de manière répétée:
a) à effectuer un rétrocroisement (backcross) de la descendance résistante à la maladie avec des plantes essentiellement homozygotes, sensibles à la maladie du taxon, et
b) à sélectionner dans la descendance du rétrocroisement les plantes qui expriment la résistance à la maladie et l'activité antimicrobienne en même temps que les autres caractéristiques souhaitées du taxon sensible, jusqu'à ce que le pourcentage souhaité des caractéristiques du taxon sensible soit présent dans la descendance en même temps que la résistance à la maladie et l'activité antimicrobienne.

12. Méthode de production d'une cellule bactérienne transformée contenant sous forme d'ADN étranger au moins une copie d'une cassette d'expression comprenant un ADN recombiné qui code sensiblement uniquement pour CMIII ayant la séquence d'acides aminés Arg-Ser-Gly-Arg-Gly-Glu-Cys -Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly, laquelle méthode comprend l'introduction de ladite cassette d'expression dans ladite cellule.

13. Méthode de production d'une cellule végétale transformée contenant sous forme d'ADN étranger au moins une copie d'une cassette d'expression comprenant un ADN recombiné qui code sensiblement uniquement pour CMIII ayant la séquence d'acides aminés Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly dans laquelle la séquence codante pour CMIII est liée de manière fonctionnelle à des séquences régulatrices de plante qui induisent l'expression de CMIII dans des cellules végétales.

14. Méthode selon la revendication 13 dans laquelle les cellules sont d'une espèce monocotylédone.

15. Méthode selon la revendication 14 dans laquelle les cellules sont des cellules de maïs, de sorgho, de blé ou de riz.

16. Méthode selon la revendication 13 dans laquelle les cellules sont d'une espèce dicotylédone.

17. Méthode selon la revendication 16 dans laquelle les cellules sont des cellules de soja, de luzerne, de tabac ou de tomate.

18. ADN recombiné qui code sensiblement uniquement pour CMIII ayant la séquence d'acides aminés Arg-Ser-Gly-Arg-Gly-Glu-Cys-Arg-Arg-Gln-Cys-Leu-Arg-Arg-His-Glu-Gly-Gln-Pro-Trp-Glu-Thr-Gln-Glu-Cys-Met-Arg-Arg-Cys-Arg-Arg-Arg-Gly-Gly.

19. ADN recombiné tel que revendiqué à la revendication 18 qui comprend une cassette d'expression dans laquelle la séquence codante pour CMIII est liée de manière fonctionnelle à des séquences bactériennes régulatrices de l'expression qui induisent l'expression de CMIII dans des cellules bactériennes.

20. Cellules bactériennes contenant sous forme d'un plasmide étranger au moins une copie d'une cassette d'expression selon la revendication 19.

21. ADN recombiné tel que revendiqué à la revendication 18 qui comprend une cassette d'expression dans laquelle la séquence codante de CMIII est liée de manière fonctionnelle à des séquences régulatrices de plante qui induisent l'expression de CMIII dans des cellules végétales.

22. Cellules de plante transformées contenant, sous forme d'ADN étranger, au moins une copie de la séquence d'ADN d'une cassette d'expression selon la revendication 21.

23. Cellules transformées selon la revendications 22 qui sont des cellules d'une espèce monocotylédone.

24. Cellules transformées selon la revendication 23 qui sont des cellules de maïs, de sorgho, de blé ou de riz.

25. Cellules transformées selon la revendication 22 qui sont des cellules d'une espèce dicotylédone.

26. Cellules transformées selon la revendications 25 qui sont des cellules de soja, de luzerne, de tabac ou de tomate.

27. Culture de cellules ou de tissu de maïs comprenant des cellules selon la revendication 24.

28. Plante transformée, dont les cellules contiennent sous forme d'ADN étranger au moins une copie de la séquence d'ADN d'une cassette d'expression selon la revendication 21.

29. Plante transformée pouvant être obtenue par une méthode selon l'une quelconque des revendications 4 à 11.

30. Plante transformée selon la revendication 28 ou 29 qui est d'une espèce telle que définie dans l'une quelconque des revendications 23 à 26.

31. Plante transformée selon la revendication 30 qui est un plant de maïs.

32. Plante transformée selon l'une quelconque des revendications 28 à 31 qui n'est pas une variété de plante.
